(19) **Europäisches Patentamt**

**European Patent Office**

**Office européen des brevets**

(11) Numéro de publication: **0 270 432 B1**

## (12) FASCICULE DE BREVET EUROPEEN

(45) Date de publication de fascicule du brevet: **20.05.92**    (51) Int. Cl.⁵: **A61F 2/02**

(21) Numéro de dépôt: **87402570.3**

(22) Date de dépôt: **16.11.87**

(54) **Dispositif filtrant pour caillots sanguins.**

(30) Priorité: **17.11.86 FR 8615962**

(43) Date de publication de la demande:
**08.06.88 Bulletin 88/23**

(45) Mention de la délivrance du brevet:
**20.05.92 Bulletin 92/21**

(84) Etats contractants désignés:
**AT BE CH DE ES FR GB GR IT LI LU NL SE**

(56) Documents cités:
**DE-A- 3 429 850
FR-A- 2 573 646
FR-A- 2 587 901
US-A- 4 425 908
US-A- 4 543 086**

(73) Titulaire: **PROMED
Baie Saint Jean
97133 Saint-Barthélemy Guadeloupe(FR)**

(72) Inventeur: **Lebigot, Jacques
Anse Toiny BP 1
F-97133 Saint-Barthélemy Guadeloupe(FR)**

(74) Mandataire: **Portal, Gérard et al
Cabinet Beau de Loménie 55, rue d'Amsterdam
F-75008 Paris(FR)**

EP 0 270 432 B1

## Description

La présente invention a pour objet un dispositif filtrant utile pour provoquer l'interruption partielle de la veine cave inférieure dans le cadre de la prévention da la maladie thrombo-embolique ; ce dispositif filtrant est destiné à être introduit dans la veine cave inférieure du corps humain, en aval du foyer emboligène pour arrêter les caillots transportés par le courant sanguin dans la veine cave de la partie inférieure du corps humain afin de barrer la route à la migration embolique vers le système circulatoire pulmonaire.

On a déjà proposé des filtres à déposer dans le système vasculaire pour arrêter les emboles.

Le brevet américain 3 540 431 décrit un filtre intravasculaire qui a une configuration en parapluie et qui comprend des bras divergents terminés en pointes et un capuchon de milieu filtrant.

Le brevet américain 3 952 747 décrit un autre filtre intravasculaire comprenant plusieurs bras divergents, réunis ensemble à l'une de leurs extrémités, et terminés chacun per un crochet à leur extrémité libre, ces bras présentant en outre des pliages en U.

Ces filtres sont positionnés dans la veine cave où ils sont maintenus en place par les pointes ou les crochets en contact avec la paroi interne de la veine. Toutefois, ils ne sont pas en équilibre stable, et il peut arriver que, sous l'effet de la circulation sanguine ou des mouvements de la veine, ces filtres basculent et se détachent de la paroi interne de la veine pour migrer dangereusement dans ladite veine et même remonter dans l'oreillette droite du coeur où ils peuvent provoquer des perforations irrémédiables.

De plus, de tels filtres ne sont pas fiables du fait que leurs mailles relativement larges peuvent laisser échapper une partie des emboles et que leur capacité de filtration est limitée.

On connaît également par le document US 4,425,908 un dispositif filtrant comportant deux filtres dont l'un est formé, dans sa forme filtrante par un jeu de boucles enchevêtrées formant une rosette, ce qui présente l'inconvénient grave d'être susceptible de conduire à un bouchage de la veine.

En outre, ce dispositif ne peut être récupéré, puisqu'il est réalisé en une matière ayant une mémoire thermique, le filtre ne pouvant être inséré dans un cathéter qu'à très basse température.

Enfin, le document DE 3 429 850 révèle un dispositif filtrant comportant trois filtres, chaque filtre étant constitué de bras pliables dont les extrémités sont en forme de pointes.

Un tel dispositif est encombrant et mal adapté pour suivre les mouvements de la veine.

En outre, il ne peut être récupéré une fois mis en place.

La présente invention a pour objet un dispositif filtrant permettant de remédier aux inconvénients des filtres classiques.

Le dispositif filtrant selon l'invention, à l'état déployé de service, comporte un corps filiforme prolongé à l'une de ses extrémités par un premier filtre constitué par plusieurs fils flexibles, de même longueur et de même configuration, partant de ladite extrémité et allant en divergeant dans le prolongement dudit corps, ces fils étant en outre maintenus à un écartement circonférentiel régulier les uns par rapport aux autres de façon à former un cône de révolution évasé et ouvert à sa base, l'extrémité libre de chaque fil étant coudée vers l'extérieur pour former une pointe d'accrochage; l'autre extrémité dudit corps est terminée par un stabilisateur faisant également office de second filtre, constitué de plusieurs fils flexibles, de même longueur et de même configuration, partant de cette autre extrémité, et repliés d'abord sur eux-mêmes autour dudit corps et allant en divergeant par rapport à ce dernier ; ces fils sont maintenus à un écartement circonférentiel régulier les uns par rapport aux autres et sont en outre décalés d'un certain angle par rapport aux fils constitutifs du premier filtre pour former un second cône de révolution évasé et ouvert à sa base ; l'extrémité libre de chaque fil est repliée légèrement vers l'intérieur pour éviter tout accrochage ; un oeillet étant également prévu à cette autre extrémité du corps pour le passage d'un fil de soie pour la mise en place du dispositif filtrant, dans la veine, et éventuellement sa récupération si celui-ci était mal positionné.

A l'état replié, pour être logé dans un cathéter. les fils du stabilisateur-filtre sont rabattus élastiquement contre ledit corps et les fils du premier filtre sont rabattus élastiquement sur l'axe qui prolonge ledit corps.

Plus particulièrement, le premier filtre est constitué par six fils flexibles maintenus à un écartement circonférentiel de 60° les uns par rapport aux autres ; le stabilisateur-filtre est constitué par six fils flexibles lesquels sont maintenus à un écartement circonférentiel de 60° les uns par rapport aux autres, et décalés d'un angle de 30° par rapport aux fils du premier filtre.

Selon un mode de réalisation particulier du dispositif filtrant selon l'invention, chacun des fils constitutifs du premier filtre est solidarisé à l'un des fils constitutifs du stabilisateur-filtre par un tronçon intermédiaire de fil pour former un élément en épingle, et un certain nombre de ces éléments en épingle est juxtaposé et solidarisé ensemble par leurs tronçons intermédiaires au moyens d'un manchon tubulaire pour reconstituer le premier filtre, le corps et le stabilisateur-filtre.

Le dispositif filtrant selon l'invention et plus particulièrement les fils flexibles qui le constituent peuvent être réalisés en un matériau élastiquement déformable quelconque, habituellement utilisé pour la fabrication des filtres intravasculaires, et de préférence en acier inoxydable de qualité médicale.

On décrit l'invention dans ce qui suit en se référant au dessin ci-joint sur lequel :

la figure 1 représente une vue en élévation latérale du dispositif filtrant selon l'invention,

la figure 2 est une vue de gauche du dispositif filtrant représenté à la figure 1,

la figure 3 représente un élément en épingle utilisé dans la fabrication dudit dispositif filtrant,

la figure 4 est une vue du dispositif filtrant à l'état replié, logé dans un cathéter,

la figure 5 est une vue du dispositif filtrant à l'état déployé, positionné dans la veine cave inférieure.

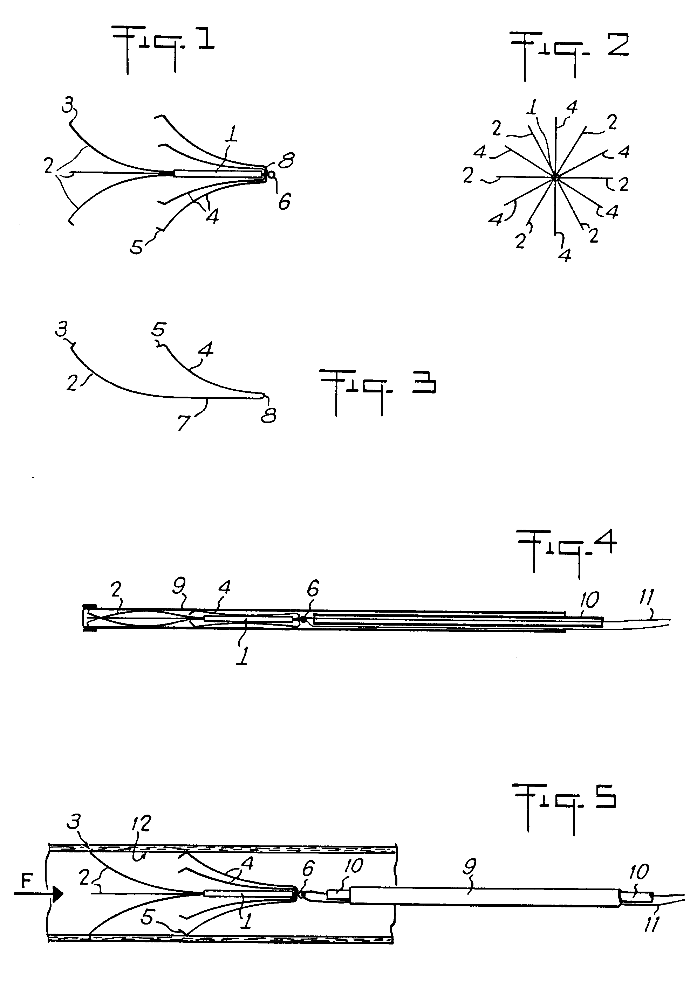

Le dispositif filtrant selon l'invention, tel que représenté en particulier aux figures 1 et 2 à l'état déployé de service, comporte un corps (1) généralement filiforme prolongé à l'une de ses extrémités par un premier filtre constitué par plusieurs fils métalliques flexibles (2) (par exemple, six fils en acier inoxydable de qualité médicale) de même longueur et de même configuration, partant de ladite extrémité et allant en divergeant dans le prolongement dudit corps, ces fils étant en outre maintenus à un écartement circonférentiel régulier les uns par rapport aux autres (par exemple à un écartement de 60°) pour former un cône de révolution évasé et ouvert à sa base ; l'extrémité libre de chaque fil est pliée à angle droit vers l'extérieur pour former une pointe (3) pour l'accrochage à la paroi interne de la veine.

L'autre extrémité du corps (1) est terminée par un stabilisateur faisant également office de second filtre, constitué de plusieurs fils métalliques (4) (par exemple, six fils en acier inoxydable de qualité médicale), de même longueur et de même configuration, partant de cette seconde extrémité, et repliés d'abord sur eux-mêmes autour dudit corps et allant en divergeant par rapport à ce dernier ; ces fils sont maintenus à un écartement circonférentiel régulier les uns par rapport aux autres, (par exemple, à un écartement de 60°) tout en étant décalés d'un certain angle (par exemple de 30°) par rapport aux fils constitutifs du premier filtre, pour former un second cône de révolution évasé et ouvert à sa base ; l'extrémité libre (5) de chaque fil est repliée ou incurvée légèrement vers l'intérieur pour éviter tout accrochage avec la paroi interne de la veine.

Un oeillet (6) est également prévu à cette seconde extrémité du corps (1) pour le passage d'un fil de soie pour la mise en place du dispositif filtrant selon l'invention dans la veine cave inférieure au moyen d'un cathéter comme on le verra ci-après.

Selon un mode de réalisation particulier du dispositif filtrant, chacun des fils (2) constitutifs du premier filtre est solidarisé à l'un des fils (4) constitutifs du stabilisateur-filtre par un tronçon intermédiaire de fil (7) pour former un élément en épingle, comme représenté à la figure 3 ; plus particulièrement, on part d'un fil métallique flexible (7), on l'incurve légèrement à l'une de ses extrémités pour former l'élément (2) et le bout de cet élément est coudé à angle droit vers l'extérieur pour former la pointe d'accrochage (3) ; l'autre extrémité du fil (7) est repliée d'abord sur elle-même parallèlement audit fil avec formation d'une boucle (8) puis incurvée vers l'extérieur pour former l'élément (4), lequel est ensuite décalé de l'élément (2) d'un angle de 30° par une légère torsion ; le bout de l'élément (4) est alors coudé vers l'intérieur.

Six éléments en épingle ainsi conformés sont solidarisés ensemble par leurs portions centrales au moyen d'un manchon tubulaire (1) pour reconstituer le premier filtre, le corps (1) et le stabilisateur-filtre ; un fil muni d'un oeillet (6) est intercalé au centre des éléments en épingle avant le sertissage du manchon.

A titre d'exemple, non limitatif, le dispositif filtrant qui vient d'être décrit est réalisé à partir d'un fil d'acier inoxydable de qualité médicale de 3/10e de millimètre de diamètre , et le manchon de sertissage également en acier inoxydable a un alésage de 1 mm de diamètre et un diamètre extérieur de 2 mm ; la longueur totale du dispositif filtrant est de 6 cm, la longueur du corps (1) est de 2,5-3 cm, la distance entre les deux filtres (distance mesurée entre les extrémités libres des fils (2) et (4)) est de 3,0 à 3,5 cm ; le diamètre de la base des cônes est de 3 cm, le rayon de courbure des fils (2) et (4) est de 3 cm.

Le dispositif filtrant selon l'invention peut être introduit par voie percutanée, our après abord chirurgical, dans la veine jugulaire interne à la base du cou, à l'aide d'un cathéter radioopaque et largué à l'endroit voulu.

A cet effet, le dispositif filtrant selon l'invention, tel que représenté à la figure 4 à l'état replié, est logé dans un cathéter (9) (par exemple de diamètre intérieur 3,2 mm, de diamètre extérieur 4 mm, et de longueur 60 cm) à l'intérieur duquel est disposée une canule (10) (par exemple de 1,2 mm de diamètre intérieur et 2,5 mm de diamètre extérieur et de 70 cm de longueur) à travers laquelle passe un fil de soie (11) enfilé dans l'oeillet (6). Un système d'assemblage du type Luer-Lock est prévu pour solidariser la canule avec le cathéter.

Comme le montrent les figures 4 et 5 le fil de soie (11) passant dans l'oeillet définit deux brins de fils dont les extrémités libres sortent du cathéter.

Ces deux brins peuvent traverser la canule (10), mais avantageusement, pour éviter qu'ils ne s'entremêlent, l'un des deux brins passe à travers la canule (10) tandis que l'autre passe à travers l'espace défini entre la canule (10) et le cathéter (9).

Le cathéter (9) est introduit dans la veine jugulaire interne à la base du cou et poussé à travers la veine cave supérieure jusque dans la veine cave inférieure.

Dès que l'emplacement désiré est atteint, on immobilise la canule (10) et on retire progressivement le cathéter (9) pour libérer le premier filtre qui se déploie dans ladite veine et ses pointes (3) viennent accrocher la paroi interne de cette veine (12). On exerce une légère traction sur le fil de soie (11) pour parfaire l'accrochage. En continuant à retirer le cathéter, on libère le stabilisateur-filtre qui se déploie et ses fils (4) viennent s'appuyer contre la paroi interne de la veine.

On retire l'ensemble cathéter (9) et canule (10) et finalement le fil de soie (11).

Si le dispositif filtrant était mal positionné, on pourrait le récupérer en poussant progressivement le cathéter (9) pour rentrer d'abord le stabilisateur-filtre, puis le premier filtre dans ledit cathéter, puis replacer le dispositif filtrant à un autre emplacement, et ceci constitue un avantage de l'invention.

Le procédé de mise en place du dispositif filtrant selon l'invention est simple et peu traumatisant, comparativement aux méthodes chirurgicales nécessaires pour la mise en place des filtres classiques, et peut être proposé à des sujets dont l'état général est très précaire. Son introduction par voie percutanée permet l'institution sans danger d'un traitement fibrinolytique. Sa mise en place peut être contrôlée par une radiographie simple de l'abdomen ou par un examen scannographique.

Ainsi positionné dans la veine cave inférieure, le dispositif filtrant selon l'invention permet d'interrompre partiellement ladite veine et de réaliser un barrage efficace contre la migration embolique, tout en n'ayant qu'un faible retentissement sur le flux veineux.

Les emboles transportés par le courant sanguin veineux dans le sens de la flèche (F) sont piégés et arrêtés par le premier filtre et ceux qui ont pu échapper à ce premier filtre en passant entre les fils (2) son arrêtés par le second filtre dont les fils (4) sont décalés d'un angle de 30° par rapport aux fils (2).

En plus du fait qu'il permet de doubler la capacité de filtration du dispositif filtrant, le stabilisateur-filtre, en raison de sa configuration en cône ouvert à sa base, qui lui confère une bonne flexibilité, n'est pas bloqué rigidement à la paroi interne de la veine, mais il peut se déformer élastiquement en suivant les mouvements de ladite veine, tout en maintenant le contact avec la paroi de cette veine et sans interférence avec le premier filtre qui demeure accroché de manière stable à la paroi interne de la veine, ce qui contribue à la fois au confort du patient et à la sécurité de l'emploi dudit dispositif filtrant.

## Revendications

1. Dispositif filtrant utile pour interrompre partiellement la veine cave inférieure et réaliser un barrage efficace contre la migration embolique comportant, dans un état déployé de service, un corps (1) filiforme,
   - prolongé à l'une de ses extrémités par un premier filtre constitué par plusieurs fils flexibles (2), de même longueur et de même configuration, partant de ladite extrémité et allant en divergeant dans le prolongement dudit corps, ces fils étant en outre maintenus à un écartement circonférentiel régulier les uns par rapport aux autres de façon à former un cône de révolution évasé et ouvert à sa base, l'extrémité libre de chaque fil étant coudée vers l'extérieur pour former une pointe d'accrochage (3),
   - l'autre extrémité dudit corps (1) étant terminée par un stabilisateur faisant également office de second filtre, constitué de plusieurs fils flexibles (4), de même longueur et de même configuration, partant de cette autre extrémité , et repliés d'abord sur eux-mêmes autour dudit corps et allant en divergeant par rapport à ce dernier, ces fils étant maintenus à un écartement circonférentiel régulier les uns par rapport aux autres et étant en outre décalés d'un certain angle par rapport aux fils constitutifs du premier filtre pour former un second cône de révolution évasé et ouvert à sa base, l'extrémité libre (5) de chaque fil étant repliée légèrement vers l'intérieur pour éviter tout accrochage, un oeillet (6) étant également prévu à cette autre extrémité du corps (1) pour le passage d'un fil de soie pour la mise en place dans la veine du dispositif filtrant, et éventuellement sa récupération si celui-ci était mal positionné et en ce que dans un état replié, pour être logé dans un cathéter, les fils (4) du Stabilisateur-filtre sont élastiquement rabattus contre le corps (1) et les fils (2) du premier filtre sont élastiquement rabattus sur l'axe qui prolonge ledit corps (1).

2. Dispositif filtrant selon la revendication 1, caractérisé en ce que le premier filtre est constitué par six fils flexibles maintenus à un écartement circonférentiel de 60° les uns par rapport aux autres, que le stabilisateur-filtre est constitué par six fils flexibles, lesquels sont maintenus à un écartement circonférentiel de 60° les uns par rapport aux autres, et décalés d'un angle de 30° par rapport aux fils du premier filtre.

3. Dispositif filtrant selon la revendication 1 ou 2, caractérisé en ce que chacun des fils (2) constitutifs du premier filtre est solidarisé à l'un des fils (4) constitutifs du stabilisateur-filtre par un tronçon intermédiaire de fil (7) pour former un élément en épingle, un certain nombre de ces éléments en épingle étant juxtaposé et solidarisé ensemble par leurs tronçons intermédiaires au moyen d'un manchon tubulaire pour reconstituer le premier filtre, le corps et le stabilisateur-filtre.

4. Dispositif filtrant selon l'une quelconque des revendications 1 à 3, caractérisé en ce que le dispositif filtrant et plus particulièrement les fils flexibles qui le constituent sont réalisés en un matériau élastiquement déformable quelconque habituellement utilisé pour la fabrication des filtres vasculaires et de préférence en acier inoxydable de qualité médicale.

5. Système pour la mise en oeuvre dans la veine du dispositif filtrant défini aux revendications 1 à 4 comprennant un cathéter (9) destiné à loger ledit dispositif filtrant dans son état replié, caractérisé en ce qu'il comprend une canule (10) logée dans ledit cathéter (9) et un fil de soie (11) s'étendant dans le cathéter (9) et destiné a passer dans l'oeillet (6) précité dudit dispositif filtrant en formant ainsi deux brins dont les extrémités libres sortent du cathéter ; ladite canule (10) étant susceptible d'être solidarisée au cathéter (9) notamment au moyen d'un système d'assemblage du type Luer-Lock.

6. Système selon la revendication 5, caractérisé en ce que l'un des brins précités du fil de soie (11) passe à travers ladite canule, tandis que l'autre passe à travers l'espace défini entre ladite canule (10) et la cathéter (9).

**Claims**

1. Filtering device useful for partly interrupting the inferior vena cava and for creating an efficient barrier against embolic migration comprising in an expanded operating condition, a filiform body (1),
   - extended at one of its ends by a first filter constituted by a plurality of flexible wires (2), of identical length and configuration, starting from said end and diverging in extension from said body, said wires being regularly circumferentially spaced apart one with respect to the other, so as to form a cone of revolution, widened and open at its base, the free end of each wire being bent outwardly to form a fastening tip (3),
   - the other end of said body (1) ending in a stabilizer which also acts as second filter, and is constituted of a plurality of flexible wires (4) of identical length and configuration, starting from said other end, and which are first bent over around said body and diverging with respect thereto, said wires being regularly circumferentially spaced apart one from the other and being, moreover, offset of a certain angle with respect to the wires constituting the first filter, in order to form a second cone of revolution widened and open at its base, the free end (5) of each wire being slightly bent inwardly to prevent any fastening and an eyelet (6) being also provided at said other end of the body (1) for threading through a silk thread for positioning the filtering device inside the vein and optionally recovering it should the device be ill inserted and in that in the collapsed state, the wires (4) of the filter-stabilizer, in order to be housed in a cathether, are resiliently bent against the body (1) and the wires (2) of the first filter are resiliently bent over the axis extending from said body (1).

2. Filtering device according to claim 1, characterized in that the first filter is constituted by six flexible wires regularly circumferentially spaced apart of 60° one with respect to the other, in that the filter-stabilizer is constituted by six flexible wires which are regularly circumferentially spaced of 60°, one with respect to the other, and which are angularly offset of 30° with respect to the wires of the first filter.

3. Filtering device according to claim 1 or 2, characterized in that each one of the wires (2) constituting the first filter is secured to one of the wires (4) constituting the filter-stabilizer by an intermediate piece of wire (7) to form a pin-like element, a certain number of such pin-like elements being juxtaposed and joined together by their intermediate pieces by means of a tubular sleeve in order to make up the first filter, the body and the filter-stabilizer.

4. Filtering device according to any one of claims 1 to 3, characterized in that the filtering device and more particularly the flexible wires which constitute it, can be produced from any elastically-deformable material, conventionally used for producing intravascular filters, and preferably in a medical-grade stainless steel.

5. System for utilizing in the vein the filtering device defined in claims 1 to 4, said system comprising a catheter (9) for housing said filtering device in collapsed condition, characterized in that it comprises a canula (10) housed in said catheter (9) and a silk thread (11) which extends through said catheter (9) and designed to pass through an eyelet (6) of said filtering device so as to form two strands, the free ends of which emerge from the catheter; said canula (10) being connectable to the catheter (9) by means of an assembling system such as of the Luer-Sock-type.

6. System according to claim 5, characterized in that one of said strands of the silk thread (11) is threaded through said canula whereas the other traverses the space defined between said canula (10) and the catheter (9).

## Patentansprüche

1. Filtervorrichtung zur teilweisen Unterbrechung der Vena cava inferior und zur Herstellung einer wirksamen Sperre gegen Emboluswanderung, welche in einem entfalteten Betriebszustand einen drahtförmigen Körper (1) umfaßt,
   - der an einem seiner Enden durch einen ersten Filter verlängert ist, welcher aus mehreren flexiblen Drähten (2) gleicher Länge und gleicher Ausgestaltung gebildet ist, die von besagtem Ende ausgehen und in der Verlängerung des Körpers divergieren, welche Drähte weiter derart in einem gleichmäßigen Umfangsabstand in bezug aufeinander gehalten sind, daß sie einen trichterförmig erweiterten und an seiner Basis offenen Kreiskegel bilden, wobei das freie Ende jedes Drahts zur Bildung einer Verhakungsspitze (3) noch außen umgebogen ist,
   - wobei das andere Ende des Körpers (1) in einem Stabilisator endet, der auch als zweiter Filter fungiert und aus mehreren flexiblen Drähten (4) gleicher Länge und gleicher Ausgestaltung gebildet ist, die von diesem anderen Ende ausgehen und zuerst über sich selbst um den Körper umgeschlagen sind und in bezug auf diesen divergieren, welche Drähte in einem gleichmäßigen Umfangsabstand in bezug aufeinander gehalten sind und weiters zur Bildung eines zweiten trichterförmig erweiterten und an seiner Basis offenen Kreiskegels um einen bestimmten Winkel zu den den ersten Filter bildenden Drähten versetzt sind, wobei das freie Ende (5) jedes Drahtes zur Vermeidung jeglichen Verhakens leicht nach Innen gebogen ist, eine Öse (6) für den Durchtritt eines Seidenfadens zum Anordnen der Filtervorrichtung in der Vene und gegebenenfalls bei schlechter Positionierung wieder dessen Entfernung ebenfalls an diesem anderen Ende des Körpers (1) vorgesehen ist, und wobei in einem gefalteten Zustand zur Anordnung in einem Katheter die Drähte (4) des Stabilisatorfilters nachgiebig gegen den Körper (1) umgebogen sind und die Drähte (2) des ersten Filters nachgiebig gegen die den Körper (1) verlängernde Achse umgeklappt sind.

2. Filtervorrichtung nach Anspruch 1, dadurch gekennzeichnet, daß der erste Filter durch sechs flexible Drähte gebildet ist, die in einem Umfangsabstand von 60° gegeneinander gehalten sind, daß der Stabilisatorfilter durch sechs flexible Drähte gebildet ist, die in einem Abstand von 60° gegeneinander gehalten und um einen Winkel von 30° zu den Drähten des ersten Filters versetzt sind.

3. Filtervorrichtung nach Anspruch 1 oder 2, dadurch gekennzeichnet, daß jeder der den ersten Filter bildenden Drähte (2) über einen Drahtzwischenabschnitt (7) mit einem der den Stabilisatorfilter bildenden Drähte (4) zur Bildung eines nadelförmigen Elements verbunden ist, wobei eine gewisse Anzahl an solchen nadelförmigen Elementen nebeneinander angeordnet und über ihre Drahtzwischenabschnitte mittels einer rohrförmigen Hülse zur Bildung des ersten Filters, des Körpers und des Stabilisatorfilters verbunden sind.

4. Filtervorrichtung nach einem der Ansprüche 1 bis 3, dadurch gekennzeichnet, daß die Filtervorrichtung und insbesondere die diese bildenden flexiblen Drähte aus irgendeinem üblicherweise bei der Herstellung von Gefäßfiltern verwendeten, elastisch verformbaren Material, vorzugsweise aus rostfreiem Stahl medizinischer Güte, hergestellt sind.

5. System zur Anwendung der Filtervorrichtung nach einem der Ansprüche 1 bis 4 in der Vene, umfassend einen Katheter (9) zur Anbringung der Filtervorrichtung in ihrem gefalteten Zustand, dadurch gekennzeichnet, daß es eine im Katheter (9) befindliche Kanüle (10) und einen entlang des Katheters (9) verlaufenden und durch die Öse (6) der Filtervorrichtung zu führenden Seidenfaden (11) umfaßt, wodurch zwei Stränge entstehen, deren freie Enden aus dem Katheter herauskommen, wobei die Kanüle (10) mit dem Katheter (9) verbunden werden kann, insbesondere mittels eines Verbindungssystems des Typs Luer-Lock.

6. System nach Anspruch 5, dadurch gekennzeichnet, daß einer der genannten Stränge des Seidenfadens (11) durch die Kanüle durchgeht, während der andere durch den zwischen der Kanüle (10) und dem Katheter (9) begrenzten Raum verläuft.

EP 0 270 432 B1

Fig. 1

Fig. 2

Fig. 3

Fig. 4

Fig. 5